# EUROPEAN PATENT SPECIFICATION

(11) **EP 0 938 589 B1**
(45) Date of publication and mention of the grant of the patent: **27.08.2003**
(21) Application number: 97945607.6
(22) Date of filing: 05.11.1997
(51) Int. Cl.: C12Q 1/68

(54) **METHOD OF IDENTIFYING PHARMACEUTICALS FROM PLANT EXTRACTS**
VERFAHREN ZUR IDENTIFIZIERUNG VON PHARMAZEUTIKA AUS PFLANZENEXTRAKTEN
PROCEDES D'IDENTIFICATION D'AGENTS PHARMACEUTIQUES A PARTIR D'EXTRAITS DE VEGETAUX

(30) Priority: 07.11.1996 US 744983
(43) Date of publication of application: 01.09.1999
(73) Proprietor: SOCIETE DE CONSEILS DE RECHERCHES ET D'APPLICATIONS SCIENTIFIQUES S.A. (S.C.R.A.S.), 75016 Paris (FR); Beaufour, Albert, London W8 5HH (GB)
(72) Inventor: BEAUFOUR, Albert, London W8 5HH (GB); MOREAU, Jacques-Pierre, Upton, MA 01568 (US)
(74) Representative: Lunt, Mark George Francis
(86) International application number: US9720103
(87) International publication number: WO98020163

(56) References cited:
- EP-A- 0 534 640
- WO-A-95/17905
- WO-A-96/24604
- US-A- 4 774 226

## Description

### Background of the Invention

The microbial, plant, and animal (e.g. invertebrates) kingdoms constitute rich natural repositories of active ingredients with varied physicochemical and medicinal properties. The U.S. and European pharmacopeias are replete with examples of medicaments derived from natural sources. See U.S. Pharmacopeia 1995 (United States Pharmacopeial Convention, Inc., 1994) and Martindale, The Extra Pharmacopeia 31 (Royal Pharmaceutical Society, 1996). Many antimicrobial agents (e.g., penicillins, cephalosporins, and aminoglycosides), antifungals (e.g., amphotericin B and nystatin), antiparasitics (e.g., quinine), cardio- and vaso-active agents (e.g., cardiac glycosides-digoxin, ergot alkaloids, nicotine, and oxytocin), anti-inflammatory agents (e.g., aspirin), muscarinic (e.g., acetylcholine) and antimuscarinic (e.g., atropine and scopolamine) agents, neuroactive agents (e.g., curare, physostigmine, and opiates), anticoagulants (e.g., heparin), antineoplastic agents (e.g., vinca alkaloids, taxol, and podophyllotoxin derivative etoposide), and hormones (e.g., estrogens, androgens, progestins, peptide hormones, and growth factors) were discovered as natural products. See, e.g., Goodman & Gilman's The Pharmacological Basis of Therapeutics (9th Edition McGraw-Hill, 1996). These examples suggest that some active components in plant extracts may also be purified and still retain their biological potency.

In most of the above examples, the salient ethnopharmacological properties of the compound were characterized in known pharmacological systems and assays only upon isolation of the compound from the natural source. See, e.g., Turner, R.A. screening Methods in Pharmacology (Academic Press, 1965) and Turner, R.A., Peter Hebborn Screening Methods in Pharmacology, (Vol. II, Academic Press, 1971). While the process of determining or substantiating activities of plant Extracts may be discovered by the screening of their individual components, many of these extracts may have additional pharmacological activities that may not be evident from the extract's known, if any, ethnopharmacological background of its individual components, (e.g., the extract's ability to affect signal transduction to the nucleus, alter the trafficking of cell surface receptors, and regulate genomic events).

The characterization of defined active natural compounds, either derived from natural sources or produced synthetically or semisynthetically, constitutes the pharmacological basis for much of Western Medicine. The process of distilling an ethnopharmacologically active plant extract down to a single active principal, however, may result in a loss of biological activity for a number of reasons (e.g., a particular compound is unstable during extraction or in the purified form, the compound may react with chemical entities in the extract during purification, the compound is fractioned out during purification, or, more importantly, the fundamental basis for ethnopharmacology does not: always reside in a single active compound being present: in the extract but rather is a result of the interaction of two or more active compounds found in the extract). Thus, the likelihood that more than one compound present in a plant extract could contribute to a net pharmacological response of the extract, as well as a genomic means to identify such compounds in a natural product, is a novel pharmacological concept.

The present invention provides for a genomic screen in which an extract from a plant may be characterized for its potential biological properties which may or may not be related to the known, if any, ethnopharmacological properties of the extract. The present invention represents a novel approach to the analysis of the potential mechanism(s) of action of plant extracts, as the underlying basis for therapeutic efficacy, and a means for identifying the individual compound(s) which elicit the discovered biological property in order to identify a new pharmaceutical or new pharmaceutical use for an existing pharmaceutical.

### Summary of the Invention

In one aspect, the invention features a method of identifying a pharmaceutical, the method comprising the steps of: administering a plant extract to a cell type; isolating protein or RNA (e.g., messenger RNA) from the plant extract treated cell type; identifying which of the protein or RNA isolated from the plant extract treated cell type is not present in the same concentration in the untreated cell type (e.g., a protein or RNA which is up-regulated, down-regulated, turned on, or turned off in the cell type by the plant extract); administering compound(s) to the cell type, wherein the compound(s) are found in the plant extract; isolating protein or RNA from the compound(s) treated cell type; and identifying which of the compound(s) also result in the expression or suppression of the protein or RNA which is not present in the same concentration in the untreated cell type.

What is meant by "plant extract" is a collection of different natural compounds which are isolated from a plant (e.g., from the leaves, bark, fruits, flowers, seeds, or roots). Examples of such an extract is an extract from the tree ginkgo biloba. What is meant by "cell type" is either an isolated cell (e.g., derived from an organism such as a human or animal) or cells contained within a tissue or an organ from an organism. The cell type may be a normal cell or a diseased cell (e.g., a cell which is pathologically or physiologically different from its normal cell type, such as a tumor cell).

The plant extract and compound(s) may be administered to the cell type either in vitro or in vivo (e.g., to an intact animal from which the cell type is subsequently derived following treatment with the plant extract or compound(s)). When administered *in vitro*, the protein or RNA, for example, may be isolated immediately following or up to a day following administration of the plant extract or compound(s). When administered *in vivo*, the protein or RNA, for example, may be isolated immediately following or up to a day following the administration of the plant extract or compounds to the animal. The protein may remain within the cell type or secreted outside the cell type. Examples of such proteins include receptors, growth factors, enzymes, or transcription factors.

In another aspect, the invention features a method of determining the genomic response of a cell type to a plant extract, the method comprising the steps of:
administering the plant extract to the cell type;
isolating protein or RNA (e.g., messenger RNA) from the plant extract treated cell type; and comparing the protein or RNA isolated from the plant extract treated cell type to protein or RNA isolated from the untreated cell type.

In one embodiment, the method further comprises the step of identifying which of the protein or RNA isolated from the plant extract treated cell type is not present in the same concentration in the untreated cell type. In a further embodiment, the method further comprises sequencing the protein, RNA, or protein encoded by the RNA, identified from the plant extract treated cell type which is not present in the same concentration in the untreated cell type. In a still further embodiment, the method further comprises identifying the gene encoding the protein or RNA identified from the plant extract treated cell type which is not present in the same concentration in the untreated cell type.

In another embodiment, the cell type is associated with a known biological target (e.g., activation site) of the plant extract. For example, if the plant extract is known to treat asthma, cancer, circulation, or neurological disorders, then the cell type used is a lung respectively.

Other features and advantages of the present invention will be apparent from the detailed description of the invention and from the claims.

### Detailed Description of the Invention

It is believed that one skilled in the art can, based on the description herein, utilise the present invention to its fullest extent. The following specific embodiments are, therefore, to be construed as merely illustrative, and not limitative of the remainder of the disclosure in any way whatsoever.

Unless defined otherwise, all technical and scientific terms used herein have the same meaning as commonly understood by one of ordinary skill in the art to which this invention belongs.

### Plant Extracts and its Individual Compounds

Plant extracts may be prepared by standard chemical extraction techniques (e.g., using water to extract hydrosoluble compounds, alcohols and acetone to extract liposoluble compounds, or mixtures thereof). The extract may then be further purified to decrease the number of compounds in the extract, or even isolate a single compound or compound class, by using standard techniques such as chromatography and crystallisation. See, e.g., Ginkgolides - Chemistry, Biology, Pharmacology and Clinical Perspectives, edited by P. Braquet (J.R. Prous, Science Publishers, Barcelona, Spain 1988); Okabe, J. Chem. Soc. (c) p. 2201 (1967); and Nakauishi, Pure & Applied Chemistry 19:89 (1967).

### Identified of Genomic Events by Isolating Protein

The target cell types, in culture, are harvested before and after exposure to a set concentration(s) of a plant extract (e.g., an extract of ginkgo biloba). The procedure may be similarly applied to cells from a target tissue or organ of an organism that has been exposed to a dose, or multiple doses, of a plant extract. The cells are lysed, and the proteins are solubilised in detergenised buffers (e.g., Tween 20™, SDS™, or NP-40™ available from Sigma Chemicals, St. Louis, MO). The proteins are separated by two-dimensional gel electrophoresis, with isoelectric focusing in the first dimension and a gradient gel electrophoresis in the second dimension. See, e.g., Ausubel, A.M., et al., Current Protocols in Molecular Biology, pp 10.3.1-10.4.5 (John Wiley & Sons, 1987). This is an extremely powerful method for examining complex mixtures of proteins (e.g., as many as 1500 proteins may be resolved in a single 2-D Gel). See, e.g., O'Farrell, P.H., J. Biol. Chem. 250:4007-4021 (1975).

The pattern of protein expression may be visualised by Coomasie Blue staining (Ausubel, A.M., et al., Current Protocols in Molecular Biology, pp 10.6.1 (John Wiley & Sons, 1987)) or silver staining (Ausubel, A.M., et al., Current Protocols in Molecular Biology, pp 10.6.1-10.6.3 (John Wiley & Sons, 1987)). The cells may also be pulsed with a radio-labelled amino acids, e.g. S³⁵-Methionine (Amershaw Corp., Arlington Heights, IL), and the pattern of labelled protein expression on the 2-D Gels is detected by autoradiography. In the case where the pattern of post-translationally modified proteins are examined, e.g., phosphorylated proteins, a radioactive phosphate donor, such as P³²-γATP (Amersham Corp., Arlington Heights, IL), is added to the incubation medium, and the pattern of phosphorylated protein on the 2-D gels may then be visualized by autoradiography. See, e.g., Hansen, K., et al., Electrophoresis 14:112-116 (1993); and Guy, R., Electrophoresis 15:417-440 (1994). In the latter case, unlabelled phosphoprotein expression may be visualized using phosphotyrosine or phosphoserine antibodies upon transfer of the gels to nitrocellulose. See, e.g., Ausubel, A.M., et al., Current Protocols in Molecular Biology, pp 10.7.1- 10.8.6 (John Wiley & Sons, 1987).

Specific genomic events are detected by comparing the pattern of protein expression in cells before and after exposure to the plant extract. Specific protein spots on the 2-D gels that show either an enhancement or a reduction in expression, as evident from the intensity of the stain on the protein map, represents changes in genomic activity induced by components in the plant extract. The method is highly reproducible and can provide a means of collecting small amounts of extremely pure proteins for amino acid sequence analysis using standard techniques (i.e., Edmans amino-terminal degradation chemistry). See, e.g., Graven, et al., J. Biol. Chelm. 269:24446 (1994).

For proteins that are expressed at low quantities wherein sufficient amounts of the material may not be obtain for peptide sequencing, the protein spots may be excised from the 2-D gel and used directly for immunization in rabbits for antibody production. See Harlow, E. & Lane, D., Antibodies: A Laboratory Manual, Cold Spring Harbor Laboratory, 1988, pp. 61. The availability of an antibody allows the immunoaffinity purification of larger amounts of the regulated protein for peptide sequence identification.

From the peptide sequence, oligonucleotides may be synthesized based on redundant or most preferred genetic code for use as probes to screen a cDNA library to obtain the full length coding sequence of the gene(s). See, e.g., Sambrook, J., et al., Molecular Cloning: A Laboratory Manual, Book 2, (2nd Edition, Cold Spring Harbor Laboratory Press, 1989).

### Identification of Genomic Events By Isolating mRNA

Another method for detecting changes in genomic events in response to a plant extract is to monitor messenger RNA (mRNA) changes in a cell (Liang, P. & Pardee, A.B., Science 257:967-971 (1992)). The cell will be exposed to a concentration of the plant extract in culture, or the cells from a target tissue or organ in an organism can be exposed locally or systemically with a dose or multiple doses of the plant extract for a defined period and subsequently extracted.

The mRNA will be prepared by routine total RNA extraction methods as described in Ausubel, A.M., et al., Current Protocols in Molecular Biology, pp 4.1.2- 4.3.4 (John Wiley & Sons, 1987). From this preparation, Poly(A⁺) RNA may be prepared by oligo-dT chromatography (Aviv, H., & Leder, P., J. Mol. Biol. 134:743 (1972)) as described in Ausubel, A.M., et al., Current Protocols in Molecular Biology, pp 4.5.1-4.5.3 (John Wiley & Sons, 1987).

Two pools of mRNA are prepared, i.e., one from cells before and the other after exposure to the plant extract. Specific genomic events are represented in both pools depending on whether a suppression of gene activity or an activation of gene expression is induced by the plant extract. In the case where a gene is down-regulated in response to the plant extract, the mRNA will be present in higher quantities in the mRNA pool derived from the control cells. In the case where a gene is up-regulated in response to the plant extract, the mRNA will be present in higher quantities in the mRNA pool obtained from the plant extract treated cells. A number of techniques may be employed to identify mRNA populations that are up-regulated or down-regulated, e.g., subtractive hybridization or differential display.

### (a) Subtraction Hybridization

Subtractive hybridization techniques (Lee, S.W., et al., Proc. Natl. Acad. Sci. 88:2825 (1991)) have been developed wherein the mRNA from one of the two pools is converted to first strand cDNA (antisense) by using oligo-dT primers, attached to either cellulose beads or free primers, and reverse transcriptase. See, e.g., Sambrook, J., et al., Molecular Cloning: A Laboratory Manual, Book 2, pp 10.46 (2nd Edition, Cold Spring Harbor Laboratory Press, 1989). In the case where the first strand is attached to cellulose beads, subtractive mRNA chromatography may be effected by passing the second mRNA pool through the column. mRNA represented in both pools will hybridize (i.e., mRNA from the second pool will hybridize to the antisense cDNA on the column and will be retained on the column). The flow through, which does not hybridize in the column, will contain mRNA species arising from an alteration in genomic activity from the effect of the plant extract on the cells. A cDNA bacteriophage library can then be prepared with mRMA from the flow-through fraction. See, e.g., Sambrook, J., et al., Molecular Cloning: A Laboratory Manual, Book 2, pp 8.11-8.45 (2nd Edition, Cold Spring Harbor Laboratory Press, 1989).

Specific clones not represented in the first mRNA pool are identified as non-hybridizing plaques if the library is screened with an P³²-end-labelled probes generated from the first pool. Other comparable strategies have also been described (Sambrook, J., et al., Molecular Cloning: A Laboratory Manual, Book 2, pp 10.41, 10.42 (2nd Edition, Cold Spring Harbor Laboratory press, 1989).

The genes involved will be identified by DNA sequencing of the cDNA clones. See, e.g., Sambrook, J., et al., Molecular Cloning: A Laboratory Manual, Book 2, pp 13.3-13.20 (2nd Edition, Cold Spring Harbor Laboratory Press, 1989).

### (b) mRNA Differential Display

This method is well described in the art. See, Liang, P., & Pardee, A.B., Science 257:967-970 (:.992); Callard, D., et al., BioTechniques 16:1096-1103 (1994); Chen, Z., et al., BioTechniques 16:1003-1006 (1994); and Zhao, S., et al., BioTechniques 20:400-404 (1996). Partial cDNA sequences from mRNA prepared from the plant extract or treated and untreated cells are prepared by reverse transcription. See, e.g., Sambrook, J., et al., Molecular Cloning: A Laboratory Manual, Book 2, pp 10.46 (2nd Edition, Cold Spring Harbor Laboratory Press, 1989) and amplified by polymerase-chain reaction, e.g., using a 5'-T₁₁CA *as* a 3' -primer and a short (6-7 base) 5' -primer. The amplification reaction employs an α³⁵S-dATP label. The labelled, short PCR-amplified fragments from each mRNA pool are then fractionated (e.g., displayed) on a DNA sequencing gels. Bands not represented in equal intensity in one pool or the other are excised from the gel, reamplified, and used as a probe for screening a cDNA library, as described above, to identify the genes that are regulated by the plant extract.

### Use

In this manner, a gene profile for a specific cell type in response to exposure to a particular plant extract of pharmacological interested may be catalogued without knowing the actual components in the plant extract. The utility of the plant extract can then be converted with the reported role of some of these genes in specific biological processes. In this manner, the Therapeutic potential of the product may be extended beyond its known ethnopharmacological properties, or its known ethnopharmacological property can be genomically verified. Furthermore, similarly acting individual compound(s) in the extract can subsequently be identified as therapeutics based on the extract's activity on a certain set of genomic events.

## Claims

1. A method of identifying a pharmaceutical, said method comprising the steps of:
administering a plant extract to a cell type; isolating protein or RNA from said plant extract treated cell type;
identifying which of said protein or RNA isolated from said plant extract treated cell type is not present in the same concentration in the untreated cell type;
administering compound(s) to said cell type, wherein said compound(s) are found in said plant extract;
isolating protein or RNA from said compound(s) treated cell type; and
identifying which of said compound(s) also result in the expression or suppression of said protein or RNA which is not present in the same concentration in said untreated cell type, whereby administration to humans is excluded.

2. A method of determining the genomic response of a cell type to a plant extract, said method comprising the steps of:
administering said plant extract to said cell type;
isolating protein or RNA from said plant extract treated cell type; and
comparing said protein or RNA isolated from said plant extract treated cell type to protein or RNA isolated from the untreated cell type, whereby administration to humans is excluded.

3. A method of claim 1 or 2, wherein said method comprises isolating protein from said plant extract treated- cell type and, in the case of the method of claim 1, from said compound(s) treated cell type.

4. A method of claim 1 or 2, wherein said method comprises isolating RNA from said plant extract treated cell type and, in the case of the method of claim 1, from said compound(s) treated cell type; and wherein said RNA is preferably messenger RNA.

5. A method of claim 1 or 2, wherein said plant extract and said compound(s) are administered in vitro; or
wherein said plant extract and said compound(s) are administered in vivo.

6. A method of claims 2 and 3, wherein said method further comprises the step of identifying which of said protein isolated from said plant extract treated cell type is not present in the same concentration in the untreated cell type.

7. A method of claim 6, wherein said method further comprises sequencing said protein identified from said plant extract treated cell type which is not present in the same concentration in said untreated cell type.

8. A method of claim 7, wherein said method further comprises identifying the gene encoding said protein identified from said plant extract treated cell type which is not present in the same concentration in said untreated cell type.

9. A method of claims 2 and 4, wherein said method further comprises the step of identifying which of said RNA isolated from said plant extract treated cell type is not present in the same concentration in the untreated cell type.

10. A method of claim 9, wherein said method further comprises sequencing said RNA identified from said plant extract treated cell type which is not present in the same concentration in said untreated cell type.

11. A method of claim 10, wherein said method further comprises identifying the gene identified by said RNA isolated from said plant extract treated cell type which is not present in the same concentration in said untreated cell type; or
wherein said method further comprises sequencing the protein encoded by said RNA identified from said plant extract treated cell type which is not present in the same concentration in said untreated cell type.

12. A method of claim, wherein said cell type is associated with a known biological target of said plant extract.

13. A method of claim 4, wherein said RNA is messenger RNA.

## Patentansprüche

1. Verfahren zum Identifizieren eines Pharmazeutikums, das die Schritte umfaßt, bei denen man
einem Zelltyp einen Pflanzenextrakt verabreicht; man aus diesem Pflanzenextrakt-behandelten Zelltyp Protein oder RNA isoliert;
man identifiziert, welches Protein oder welche RNA, das/die aus dem Pflanzenextrakt-behandelten Zelltyp isoliert ist, nicht in der selben Konzentration in dem unbehandelten Zelltyp vorliegt;
man diesem Zelltyp Verbindung(en) verabreicht, wobei die Verbindung(en) in diesem Pflanzenextrakt gefunden werden;
man aus dem mit Verbindung(en) behandelten Zelltyp Protein oder RNA isoliert; und man
identifiziert, welche der Verbindung(en) auch in der Expression oder Suppression des Proteins oder der RNA resultiert, das/die nicht in der selben Konzentration in dem unbehandelten Zelltyp vorhanden ist, wobei die Verabreichung an Menschen ausgeschlossen ist.

2. Verfahren zur Bestimmung der genomischen Antwort eines Zelltyps auf ein Pflanzenextrakt, das die Schritte umfaßt, bei denen man
dem Zelltyp den Pflanzenextrakt verabreicht;
man aus dem Pflanzenextrakt-behandelten Zelltyp Protein oder RNA isoliert; und man
das Protein oder die RNA, das/die aus dem Pflanzenextrakt-behandelten Zelltyp isoliert ist, mit Protein oder RNA vergleicht, das/die aus dem unbehandelten Zelltyp isoliert ist, wobei die Verabreichung an Menschen ausgeschlossen ist.

3. Verfahren nach Anspruch 1 oder 2, das das Isolieren von Protein aus dem Pflanzenextrakt-behandelten Zelltyp und, im Fall des Verfahrens nach Anspruch 1, aus dem Verbindung(en)-behandelten Zelltyp umfaßt.

4. Verfahren nach Anspruch 1 oder 2, das das Isolieren von RNA aus dem Pflanzenextrakt-behandelten Zelltyp und, im Fall des Verfahrens nach Anspruch 1, aus dem Verbindung(en)-behandelten Zelltyp umfaßt.

5. Verfahren nach Anspruch 1 oder 2, bei dem man den Pflanzenextrakt und die Verbindung(en) in vitro verabreicht; oder bei dem man den Pflanzenextrakt und die Verbindung(en) in vivo verabreicht.

6. Verfahren nach Anspruch 2 und Anspruch 3, wenn abhängig von Anspruch 2, das ferner den Schritt umfaßt, bei dem man identifiziert, welches aus dem Pflanzenextrakt-behandelten Zelltyp isolierte Protein nicht in der selben Konzentration in dem unbehandelten Zelltyp vorliegt.

7. Verfahren nach Anspruch 6, das ferner das Sequenzieren des aus dem Pflanzenextrakt-behandelten Zelltyp identifizierten Proteins, das nicht in der selben Konzentration in dem unbehandelten Zelltyp vorliegt, umfaßt.

8. Verfahren nach Anspruch 7, das ferner das Identifizieren des Gens, das für das aus dem Pflanzenextrakt-behandelten Zelltyp identifizierte Protein, das nicht in der selben Konzentration in dem unbehandelten Zelltyp vorliegt, umfaßt.

9. Verfahren nach Anspruch 2 und Anspruch 4, wenn abhängig von Anspruch 2, das ferner den Schritt umfaßt, bei dem man identifiziert, welche der aus dem Pflanzenextrakt-behandelten Zelltyp isolierten RNA nicht in der selben Konzentration in dem unbehandelten Zelltyp vorliegt.

10. Verfahren nach Anspruch 9, das ferner das Sequenzieren der aus dem Pflanzenextrakt-behandelten Zelltyp identifizierten RNA, die nicht in der selben Konzentration in dem unbehandelten Zelltyp vorliegt, umfaßt.

11. Verfahren nach Anspruch 10, das ferner das Identifizieren des Gens umfaßt, das durch die aus dem Pflanzenextrakt-behandelten Zelltyp isolierte RNA, die nicht in der selben Konzentration in dem unbehandelten Zelltyp vorliegt, identifiziert ist; oder
das ferner das Sequenzieren des Proteins umfaßt, das durch die aus dem Pflanzenextrakt-behandelten Zelltyp identifizierte RNA, die nicht in der selben Konzentration in dem unbehandelten Zelltyp vorliegt, kodiert wird.

12. Verfahren nach Anspruch, bei dem der Zelltyp mit einem bekannten biologischen Target des Pflanzenextrakts assoziiert ist.

13. Verfahren nach Anspruch 4, bei dem die RNA messenger RNA ist.

## Revendications

1. Procédé d'identification d'un agent pharmaceutique, ledit procédé comprenant les étapes consistant à :
administrer un extrait végétal à un type cellulaire ; les protéines ou les ARN à partir dudit type cellulaire traité avec un extrait végétal ;
identifier celles desdites protéines ou desdits ARN isolés à partir dudit type cellulaire traité avec un extrait végétal ne sont pas présents à la même concentration dans le type cellulaire non traité ;
administrer un ou des composés audit type cellulaire, le ou lesdits composé(s) étant présent(s) dans ledit extrait végétal ;
isoler les protéines ou les ARN à partir du type cellulaire traité par le ou lesdits composés ; et
identifier lequel du ou desdits composés aboutit également à l'expression ou la suppression desdites protéines ou desdits ARN ne sont pas présents à la même concentration dans ledit type cellulaire non traité dans lequel l'administration aux humains est exclue.

2. Procédé de détermination de la réponse génomique d'un type cellulaire à un extrait végétal, ledit procédé comprenant les étapes consistant à :
administrer ledit extrait végétal audit type cellulaire ;
isoler les protéines ou les ARN à partir dudit type cellulaire traité avec un extrait végétal ; et
comparer lesdites protéines ou lesdits ARN isolés à partir dudit type cellulaire traité avec un extrait végétal avec les protéines ou les ARN isolés à partir du type cellulaire non traité dans lequel l'administration aux humains est exclue.

3. Procédé selon la revendication 1 ou 2, ledit procédé comprenant l'isolement des protéines à partir dudit type cellulaire traité avec un extrait végétal et, dans le cas de la méthode de la revendication 1, à partir du type cellulaire traité avec le ou lesdits composés.

4. Procédé selon la revendication 1 ou 2, ledit procédé comprenant l'isolement des ARN à partir dudit type cellulaire traité avec l'extrait végétal et, dans le cas de la méthode de la revendication 1, à partir du type cellulaire traité avec le ou lesdits composés.

5. Procédé selon la revendication 1 ou 2, dans lequel ledit extrait végétal et le ou lesdits composés sont administrés in vitro ; ou dans lequel ledit extrait végétal et le ou lesdits composés sont administrés in vivo.

6. Procédé selon la revendication 2 et la revendication 3 lorsque celle-ci est dépendante de la revendication 2, ladite méthode comprenant en outre l'étape consistant à identifier laquelle desdites protéines isolées à partir dudit type cellulaire traité par un extrait végétal n'est pas présente à la même concentration. dans le type cellulaire non traité.

7. Procédé selon la revendication 6, ledit procédé comprenant en outre le séquençage de ladite protéine identifiée à partir dudit type cellulaire traité par l'extrait végétal qui n'est pas présente à la même concentration dans ledit type cellulaire non traité.

8. Procédé selon la revendication 7, ledit procédé comprenant en outre l'identification du gène codant ladite protéine identifiée à partir dudit type cellulaire traité par l'extrait végétal qui n'est pas présente à la même concentration dans ledit type cellulaire non traité.

9. Procédé selon la revendication 2 et la revendication 4 lorsqu'elle dépend de la revendication 2 , ledit procédé comprenant en outre l'étape consistant à identifier lequel desdits ARN isolés à partir dudit type cellulaire traité par un extrait végétal n'est pas présent à la même concentration dans ledit type cellulaire non traité.

10. Procédé selon la revendication 9, ledit procédé comprenant en outre le séquençage dudit ARN identifié à partir dudit type cellulaire traité par un extrait végétal qui n'est pas présent à la même concentration dans ledit type cellulaire non traité.

11. Procédé selon la revendication 10, ledit procédé comprenant en outre l'identification du gène identifié par ledit ARN isolé à partir dudit type cellulaire traité par un extrait végétal qui n'est pas présent à la même concentration dans ledit type cellulaire non traité ; ou ledit procédé comprenant en outre le séquençage de la protéine codée par ledit ARN identifié à partir dudit type cellulaire traité par un extrait végétal qui n'est pas présent à la même concentration dans ledit type cellulaire non traité.

12. Procédé selon la revendication 2, dans lequel ledit type cellulaire est associé à une cible biologique connue dudit extrait végétal.

13. Procédé selon la revendication 4, ledit ARN étant un ARN messager.
